# EUROPEAN PATENT APPLICATION

(11) **EP 3 330 260 A1**
(43) Date of publication of application: **06.06.2018**
(21) Application number: 16382582.1
(22) Date of filing: 01.12.2016
(51) Int. Cl.: C07D 303/12

(54) **PROCESS FOR THE PREPARATION OF AN INTERMEDIATE FOR THE SYNTHESIS OF I.A. CARFILZOMIB**

(71) Applicant: Enantia, S.L., 08028 Barcelona (ES)
(72) Inventor: Pastó Aguilà, Mireia, 08028 BARCELONA (ES); Écija Queralt, Marta, 08031 BARCELONA (ES)
(74) Representative: ZBM Patents - Zea, Barlocci & Markvardsen

(57) **Abstract**

The present invention provides a compound which is the trichloroacetic acid salt of a compound of Formula (II) wherein R is selected from the group consisting of H, CH₃-, (CH₃)₂CH-, (CH₃)₂CHCH₂-, CH₃CH₂CH(CH₃)-, and PhCH₂-, and a process for its preparation.

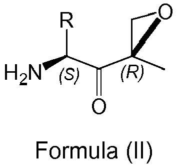

Also provided is a process for its preparation based on the stereoselective epoxidation of the corresponding olefin precursor and a process for the preparation of several pharmaceutically active ingredients comprising the preparation of the compound of formula (II).

## Description

The present invention relates to a process for the preparation of amino keto-epoxides, particularly of an intermediate for the synthesis of carfilzomib. The invention also relates to a process for the preparation of a pharmaceutically active compound such as carfilzomib from said intermediate.

### Background Art

The proteasome inhibitors are drugs that block the action of proteasomes, cellular complexes responsible for the degradation of certain proteins. Some second generation proteasome inhibitors, including carfilzomib, contain a C-terminal epoxy group. Such compounds have proven useful for the treatment of cancer, particularly multiple myeloma.

WO2006017842 and WO2005105827 disclose the preparation of carfilzomib from the trifluoroacetic acid salt of compound of Formula (IIa):

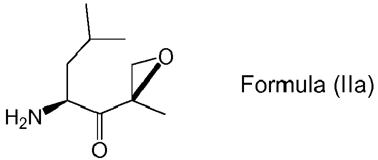

A process for the preparation of compound of Formula (IIa) comprises the epoxidation of the corresponding *N*-protected amino keto-alkene with H₂O₂ as described in N. Sin, et al. "Total synthesis of the potent proteasome-inhibitor epoxomicin: a useful tool for understanding proteasome biology", Bioorg. Med. Chem. Lett. 1999, Vol. 9, pp. 2283-2288. However, the reaction occurs with a low diastereoselectivity of 1.7:1.

WO2009045497 discloses a method of synthesis of *N*-protected amino keto-epoxides by stereoselective epoxidation of the corresponding *N*-protected amino keto-alkene with an aqueous solution of sodium hypochlorite or calcium hypochlorite in the presence of a cosolvent. It has been found (Comparative Example 2 herein below) that, following this methodology, the diastereoselectivity obtained is also low (2.4:1). The obtained N-protected amino keto-epoxide is subsequently deprotected to obtain a salt of the compound of Formula (IIa).

WO2005111009 discloses the stereoselective preparation of *N*-protected amino keto-epoxides from the corresponding allylic alcohols. However, the method involves two additional steps and the diastereoselectivity is also poor.

The diastereoselectivity of the epoxidation processes disclosed in the prior art documents mentioned above is low. The deprotection of the obtained *N*-protected amino keto-epoxide is usually carried out by the use of trifluoroacetic acid. However, in order to obtain the corresponding trifluoroacetic acid salt with a relatively good diastereomeric purity, the *N-*protected amino keto-epoxide must be previously purified by chromatography. Furthermore, the trifluoroacetic acid salt is isolated in the form of an oil, requiring an additional treatment to obtain a solid product.

Therefore, from what is known in the art, it is evident that the provision of an efficient and industrially scalable process for the preparation of a salt of the compound of formula (IIa) would be of great interest and very useful for the preparation of some pharmaceutically active compounds such as carfilzomib.

### Summary of Invention

The inventors have found that the preparation of the trichloroacetic acid salt of some amino keto-epoxides, namely of compounds of Formula (II) as defined below, significantly improves their diastereomeric purity. The trichloroacetic acid salt of the compound of Formula (II) is obtained by treatment with trichloroacetic acid of crude reaction mixtures of low diastereomeric purity of the compound of Formula (III) as defined below. Additionally, the trichloroacetic acid salt of compounds of Formula (II) precipitates in the reaction medium as it is formed, which allows the isolation of a crystalline product with an increased diastereomeric purity. Such amino keto-epoxides are precursors of some proteasome inhibitors, such as epoxomicin, carfilzomib, and other compounds such as those described in WO201136905, WO2007149512, and M. Elofsson, et al. "Towards subunit-specific proteasome inhibitors: synthesis and evaluation of peptide alpha', beta'-epoxyketones" Chemistry & Biology, 1999, Vol 6, pp.811-822. As mentioned above, known procedures to obtain said intermediates show a very low diastereoselectivity and/or require more reaction steps which further decreases yield and increases production costs.

Accordingly, an aspect of the invention relates to a compound which is the trichloroacetic acid salt of a compound of Formula (II) 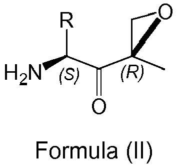 wherein R is selected from the group consisting of H, CH₃-, (CH₃)₂CH-, (CH₃)₂CHCH₂-, CH₃CH₂CH(CH₃)-, and PhCH₂-.

Another aspect of the invention relates to a process for the preparation of the trichloroacetic acid salt of a compound Formula (II) as defined above, the process comprising deprotecting the amino group of a compound of Formula (III) 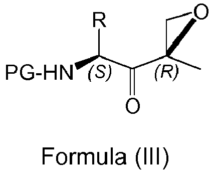 wherein R is as defined above, and PG is a protecting group selected from the group consisting of *tert*-butoxycarbonyl (Boc) and benzyloxycarbonyl (Cbz);
wherein the deprotection is carried out in the presence of the necessary amount of trichloroacetic acid to form the salt. The necessary amount of trichloroacetic acid to form the salt of the compound of Formula (II) by N-Boc cleavage is from 3 to 10 equivalents, and by *N*-CBz cleavage is from 1 to 5 equivalents.

A process as defined above, further comprising a previous step of reacting a compound of Formula (IV) 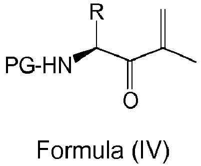 wherein R and PG are as defined above, with benzonitrile and either aqueous H₂O₂ or H₂O₂-urea in the presence of from 0.5 to 1 equivalents of a tetra(C₁-C₆)alkylammonium hydroxide as a base, in a polar solvent system, and at a suitable temperature, in order to obtain the compound of Formula (III) as defined above, also forms part of the invention.

The inventors have also found a new process for the preparation of the *N*-protected amino keto-epoxides of Formula (III) which provides the desired diastereomer (*S*,*R*) with high diastereoselectivity and good optical purity. Surprisingly, the use of a tetraalkylammonium hydroxide as a base in the epoxidation reaction of a compound of Formula (IV) as defined above with benzonitrile and either aqueous H₂O₂ or H₂O₂ urea complex, in a polar solvent system and at a suitable temperature provides the desired amino keto-epoxide with a significantly improved diastereoselectivity compared with the prior art procedures, while also obtaining a good enantiomeric excess.

Accordingly, another aspect of the present invention relates to a process for the preparation of a compound of Formula (III) 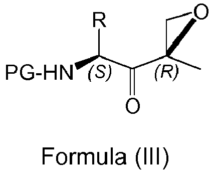 wherein R is selected from the group consisting of H, CH₃-, (CH₃)₂CH-, (CH₃)₂CHCH₂-, CH₃CH₂CH(CH₃)-, and PhCH₂;
PG is a protecting group selected from the group consisting of *tert*-butoxycarbonyl (Boc) and benzyloxycarbonyl (Cbz);
the process comprising the stereoselective epoxidation of a compound of Formula (IV) 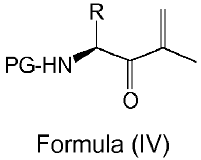 wherein R and PG are as defined above, by reaction with benzonitrile and either aqueous H₂O₂ or H₂O₂-urea in the presence of from 0.5 to 1 equivalents of a tetra(C₁-C₆)alkylammonium hydroxide as a base, in a polar solvent system, and at a suitable temperature.

Also part of the invention is a process as defined above further comprising deprotecting the amino group of the compound of Formula (III) to obtain a salt of the amino keto-epoxide of Formula (II) 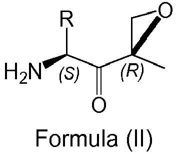 wherein R is selected from the group consisting of H, CH₃-, (CH₃)₂CH-, (CH₃)₂CHCH₂-, CH₃CH₂CH(CH₃)-, and PhCH₂;
wherein the deprotection is carried out in the presence of a necessary amount of an acid selected from the group consisting of trifluoroacetic acid, and trichloroacetic acid to yield the corresponding salt.

Another aspect of the invention relates to a process as defined above, further comprising reacting the salt of compound of Formula (II) wherein R is (CH₃)₂CHCH₂- or PhCH₂- with a peptide compound to obtain a pharmaceutically active compound selected from 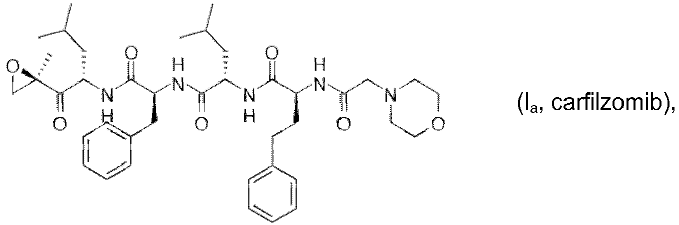 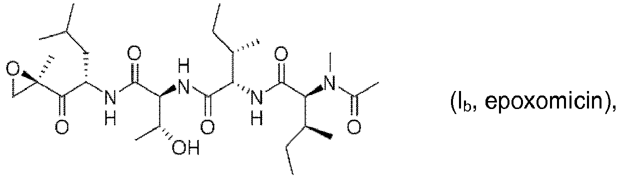 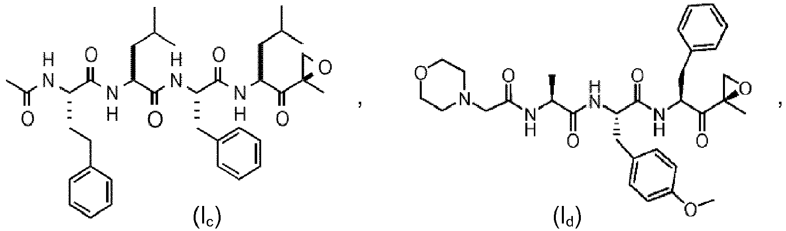 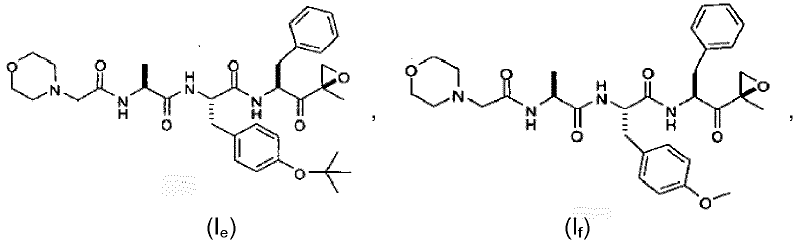 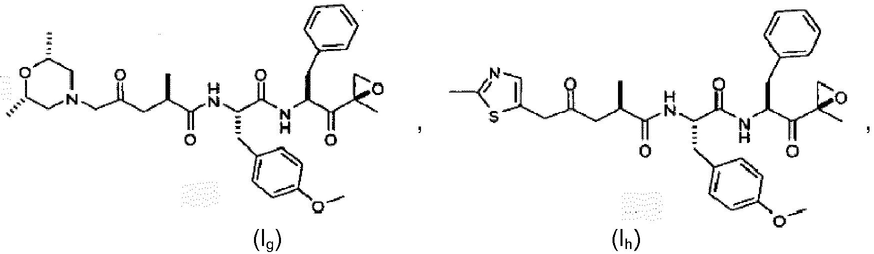 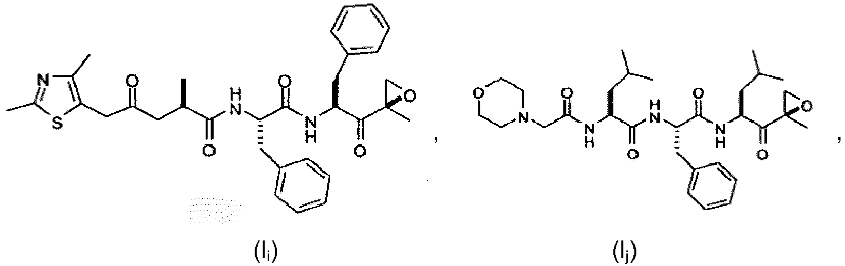 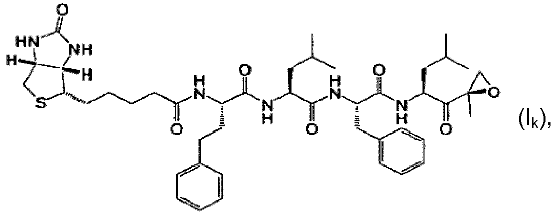 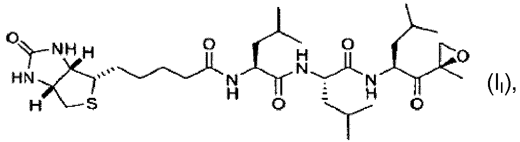 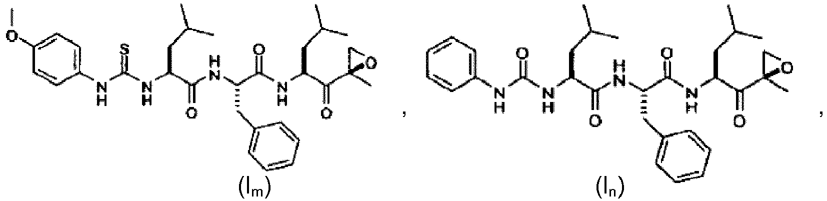 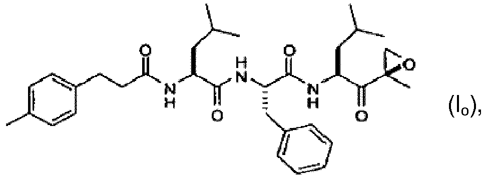 and 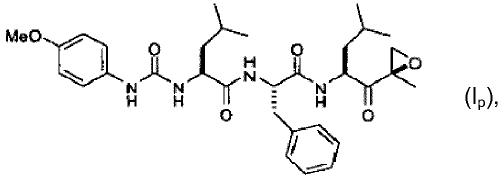 or a pharmaceutically acceptable salt thereof and, optionally, converting the pharmaceutically active compound into a pharmaceutically acceptable salt thereof by treatment of the corresponding base with a pharmaceutically acceptable acid, or converting a salt of the pharmaceutically active compound to another salt of the pharmaceutically active compound by ion exchange.

Thus, also forming part of the invention is a process for the preparation of a pharmaceutically active compound selected from those above, or a pharmaceutically acceptable salt thereof, the process comprising the steps for the preparation of a compound of Formula (II) as defined above wherein R is (CH₃)₂CHCH₂- or PhCH₂-, or a salt thereof, and reacting it with the corresponding peptide compound, and, optionally, converting the pharmaceutically active compound into a pharmaceutically acceptable salt thereof by reaction of the corresponding base with an appropriate pharmaceutically acceptable acid, or converting a salt of the pharmaceutically active compound to another salt of the pharmaceutically active compound by ion exchange.

### Brief Description of Drawings

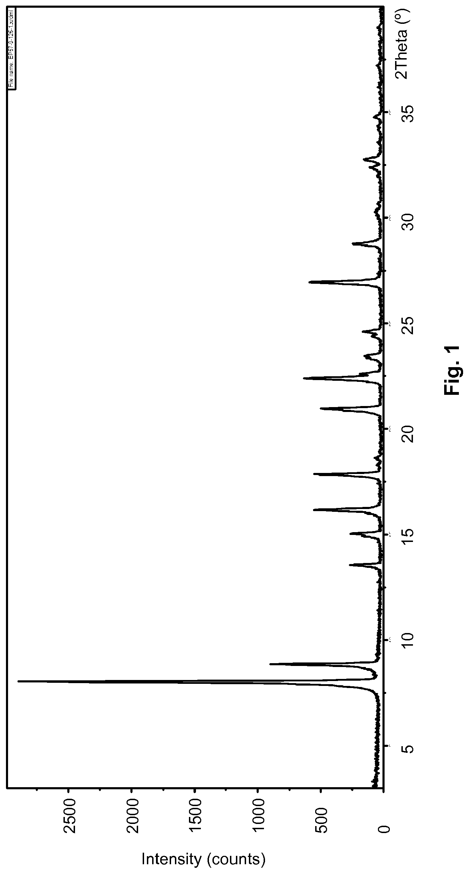

Fig. 1 shows the XRPD pattern of the crystalline form of (*S*)-2-amino-4-methyl-1-((*R*)-2-methyloxiran-2-yl)pentan-1-one trichloroacetic acid salt obtained in Example 4.

### Detailed description of the invention

All terms used herein in this application, unless otherwise stated, shall be understood in their ordinary meaning as known in the art. Other more specific definitions for certain terms used in the present application are as set forth below and are intended to apply uniformly throughout the specification and claims unless an otherwise expressly set out definition provides a broader definition.

The term "pharmaceutically acceptable salt" of a compound embraces any salt that possesses the desired pharmacological activity of the parent compound and that is formed from non-toxic pharmaceutically acceptable acids, including inorganic acids and organic acids.

Similar to carfilzomib, some of the pharmaceutically active compounds mentioned above are basic compounds. Therefore, their salts can be prepared from non-toxic pharmaceutically acceptable acids, including inorganic and organic acids. Such acids include for example acetic, benzenesulfonic, benzoic, camphorsulfonic, citric, ethanesulfonic, fumaric, gluconic, glutamic, hydrobromic, hydrochloric, lactic, phosphoric, succinic, sulfuric, tartaric, maleic, malic, mandelic, methanesulfonic, p-toluenesulfonic, and chloroacetic acids.

The preparation of pharmaceutically acceptable salts can be carried out by methods known in the art. For example, they can be prepared from the parent compound which contains a basic or acidic moiety, by conventional chemical methods. Generally, such salts are prepared, for example, by reacting the free acid or base form of these compounds with a stoichiometric amount of an appropriate pharmaceutically acceptable base or acid in water, or in an organic solvent, or a mixture thereof.

As mentioned above, one aspect of the invention relates to a compound which is the trichloroacetic acid salt of the compound of Formula (II) as defined above.

In a particular embodiment R is PhCH₂-. In another particular embodiment R is (CH₃)₂CHCH₂- and the compound is (*S*)-2-amino-4-methyl-1-((*R*)-2-methyloxiran-2-yl)pentan-1-one trichloroacetic acid salt. This trichloroacetic acid salt precipitates in the reaction medium as it is formed, which allows the isolation of a crystalline product with an increased diastereomeric purity. Thus, in a more particular embodiment, the (*S*)-2-amino-4-methyl-1-((*R*)-2-methyloxiran-2-yl)pentan-1-one trichloroacetic acid salt is in a crystalline form. In an even more particular embodiment, the crystalline form is characterized by a powder X-ray diffraction (XRPD) pattern comprising diffraction peaks at approximately 8.0, 8.9, 13,6, 15.1, 16.2, 17.9, 20.9, 22.4, 26.9, 28.8 ± 0.2 degrees 2Theta.

More particularly the crystalline form of (*S*)-2-amino-4-methyl-1-((*R*)-2-methyloxiran-2-yl)pentan-1-one trichloroacetic acid salt is characterized by a powder X-ray diffraction pattern, which is shown in Table 1 and Fig. 1:

**Table 1**

| Pos. [°2Th.] | Rel. Int. [%] |
|---|---|
| 8,0565 | 100,00 |
| 8,8851 | 29,30 |
| 13,5730 | 8,02 |
| 15,0593 | 7,94 |
| 16,1855 | 18,28 |
| 17,8527 | 18,23 |
| 20,9658 | 16,05 |
| 22,4055 | 21,64 |
| 22,6307 | 4,65 |
| 23,4792 | 4,01 |
| 24,3814 | 2,44 |
| 24,6196 | 5,05 |
| 26,9629 | 19,91 |
| 28,7650 | 7,84 |
| 32,3796 | 2,53 |
| 32,7474 | 4,38 |

XRPD analysis is obtained using a PANalytical X'Pert diffractometer with Cu-K_{α1} radiation in Bragg-Brentano geometry. The system is equipped with a monodimensional, real time multiple strip detector.

The trichloroacetic acid salt is particularly advantageous from an industrial point of view as it can be readily obtained and isolated as it filters very well, and is easily handled.

As mentioned above, the trichloroacetic acid salt of compound Formula (II) as defined above can be prepared by a process comprising deprotecting the amino group of a compound of Formula (III) as defined above in the presence of the necessary amount of trichloroacetic acid to form the salt.

In a more particular embodiment, optionally in combination with one or more features of the particular embodiments defined above or below, in the compound of Formula (III) PG is Boc and the deprotection reaction is carried out with trichloroacetic acid in a suitable solvent, such as toluene or CH₂Cl₂.

In another particular embodiment, optionally in combination with one or more features of the particular embodiments defined above or below, in the compound of Formula (III) PG is Cbz and the deprotection reaction is carried out under conditions of hydrogenolysis in the presence of trichloroacetic acid.

As mentioned above, the trichloroacetic acid salt of a compound Formula (II) as defined above can be prepared by deprotection of the amino group of a compound of Formula (III) as defined above in the presence of the necessary amount of trichloroacetic acid. In a particular embodiment, crystalline (*S*)-2-amino-4-methyl-1-((*R*)-2-methyloxiran-2-yl)pentan-1-one trichloroacetic acid salt can be prepared by a process comprising: a) crystallizing (*S*)-2-amino-4-methyl-1-((*R*)-2-methyloxiran-2-yl)pentan-1-one trichloroacetic acid salt from a solution of said compound in a solvent system comprising *tert-*butyl methyl ether (TBME), TBME/heptane, or toluene; b) isolating (i.e. by filtration) the crystalline form of (*S*)-2-amino-4-methyl-1-((*R*)-2-methyloxiran-2-yl)pentan-1-one trichloroacetic acid salt obtained in the prior step; and c) removing the solvent from the crystalline form of (*S*)-2-amino-4-methyl-1-((*R*)-2-methyloxiran-2-yl)pentan-1-one trichloroacetic acid salt obtained in step b), to yield the crystalline form as defined above.

In a more particular embodiment, the solvent used in the deprotection of the amino group of a compound of Formula (III) carried out in the presence of trichloroacetic acid is toluene and the crystalline form of (*S*)-2-amino-4-methyl-1-((*R*)-2-methyloxiran-2-yl)pentan-1-one trichloroacetic acid salt is obtained by partial evaporation of the toluene.

In another particular embodiment, the solvent used in the deprotection of the amino group of a compound of Formula (III) carried out in the presence of trichloroacetic acid is CH₂Cl₂ and the crystalline form of (*S*)-2-amino-4-methyl-1-((*R*)-2-methyloxiran-2-yl)pentan-1-one trichloroacetic acid salt is obtained by removing the solvent and adding to the obtained residue a solvent selected from the group consisting of TBME, TBME/heptane, and toluene.

As mentioned above, stereoselective epoxidation of an olefin of Formula (IV) 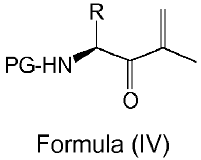 wherein R and PG are as defined above, by reaction with benzonitrile and either aqueous H₂O₂ or H₂O₂-urea in the presence of from 0.5 to 1 equivalents of a tetra(C₁-C₆)alkylammonium hydroxide or a benzyl tri(C₁-C₆) alkylammonium hydroxide as a base, in a polar solvent system, and at a suitable temperature provides the desired amino keto-epoxide with a significant increase in diastereoselectivity compared with the prior art procedures, while also obtaining a good enantiomeric excess. The reaction is carried out at a suitable temperature, which can be from 0 °C to -10 °C. Particularly, the reaction is carried out at -10 °C.

In a particular embodiment, optionally in combination with one or more features of the particular embodiments defined above or below, in the compound of Formula (IV), and consequently also in the compounds of formulas (III) and (II), R is (CH₃)₂CHCH₂-(derivative of amino acid leucine) or PhCH₂- (derivative of amino acid phenylalanine). More particularly, R is (CH₃)₂CHCH₂-.

Examples of tetra(C₁-C₆)alkylammonium hydroxides include, without being limited to, tetramethylammonium hydroxide, tetraethylammonium hydroxide, tetrabutylammonium hydroxide, and combinations thereof. Particularly, the tetra(C₁-C₆)alkylammonium hydroxide is tetramethylammonium hydroxide or tetrabutylammonium hydroxide. Examples of benzyl tri(C₁-C₆)alkylammonium hydroxide include, without being limited to, benzyltrimethylammonium hydroxide.

Examples of polar solvent systems include, without being limited to, methanol, ethanol, propanol, isopropyl alcohol, butanol, *tert*-butanol, dimethylformamide, tetrahydrofuran, dimethylformamide, dimethylsulfoxide, acetonitrile, or a mixture thereof, optionally in the presence of water. Particularly, the polar solvent is methanol.

In another particular embodiment, optionally in combination with one or more features of the particular embodiments defined above or below wherein the amount of benzonitrile is from 1 to 3 equivalents and the amount of H₂O₂ or H₂O₂-urea in the form of an aqueous solution is from 3 to 6 equivalents. [claim 11] Particularly, the reaction is carried out for 8 to 24 hours.

The protected amino keto-olefin of Formula (IV) is prepared according to the procedure described in WO2009045497 (see "Synthesis of (J)"). Particularly, it is obtained by reaction of a Weinreb amide precursor with isopropenyl magnesium bromide in tetrahydrofuran. This document specifically discloses the preparation of the mentioned olefin wherein the protecting group (PG) is a tert-butyloxycarbonyl (Boc) group. However, the use of other amine protecting groups such as benzyloxycarbonyl (Cbz), and the corresponding coupling and deprotection reagents are well known in the art of peptide synthesis and can be applied in the preparation of the intermediates described herein (see for example TW Green et al., "Protective Groups in Organic Chemistry", chapter 7, "Protection for the amino Group ", Wiley, 3rd ed. 1999, pp. 503-550). In a particular embodiment, optionally in combination with one or more features of the particular embodiments defined above or below, the protective group (PG) is selected from Boc and Cbz. More particularly, PG is Boc.

The enantiomer of the compound of Formula (IV) can be similarly obtained by starting from the amino acid of opposite stereochemistry. Then, by carrying out the process of the invention the corresponding enantiomers of compounds of Formula (III) as defined above and the corresponding enantiomers of the salt of compound of Formula (II) as defined above are obtained.

As discussed above and as shown in the examples, the use of a tetraalkylammonium hydroxide as a base in the epoxidation reaction of the olefin of Formula (IV) with benzonitrile and either aqueous H₂O₂ or H₂O₂ urea complex, in a polar solvent system and at a suitable temperature allows obtaining particularly high diastereoselectivities with good optical purities. Advantageously, the analysis of the reaction crude by ¹H-NMR shows that the compound of Formula (III) as defined above is obtained with a diastereomeric ratio equal to or higher than 8:1, particularly equal to or higher than 11:1, equal to or higher than 14:1, or equal to or higher than 20:1, of the (*S*,*R*) diastereomer versus the (*S,S*) diastereomer. This allows isolating the desired product (the (*S*,*R*) diastereomer of the compound of Formula (III)) with moderate yields, particularly from 30% to 40% after chromatographic purification.

The deprotection reaction, i.e. the removal of the amine protecting group, can be carried out by conventional methods known to those skilled in the art. The deprotection reagents and conditions depend on the particular protecting group to be removed and can be found in T. W. Green et al. (Ibid*.*). Thus, for example, for the removal of the Boc protecting group an acid such as trifluoroacetic acid or trichloroacetic can be used. The removal of the Cbz protecting group can be performed under conditions of hydrogenolysis.

As mentioned above, as an alternative to chromatographic purification, the compound of Formula (III) can be deprotected by treatment of the reaction crude with the appropriate reactants to obtain the amino keto-epoxide of Formula (II) in the form of a salt, which subsequently can be crystallized and isolated to obtain the salt of the mentioned (*S,R*)-amino keto-epoxide with an even higher diastereomeric purity.

Surprisingly, as mentioned above, the inventors have found that when deprotection of the amino group is carried out in the presence of trichloroacetic acid, the corresponding trichloroacetic acid salt of compound of Formula (II) as defined above can be directly obtained from the crude containing the corresponding compound of Formula (III) and can be isolated by crystallization and filtration with an even higher diastereomeric purity while maintaining a good optical purity. Particularly, the crystalline trichloroacetic acid salt of the compound of Formula (II) wherein R is (CH₃)₂CHCH₂-, namely crystalline (*S*)-2-amino-4-methyl-1-((*R*)-2-methyloxiran-2-yl)pentan-1-one trichloroacetic acid salt as defined above, can be obtained with a diastereomeric ratio equal to or higher than 28:1, and an enantiomeric ratio equal to of higher than 96:4. As a consequence, the diastereomeric purity can be significantly improved even starting from a reaction crude containing the corresponding compound of Formula (III) with a very low diastereomeric purity. Thus, advantageously, by the preparation of the trichloroacetic acid salt of a compound of Formula (II) as defined above, no chromatographic purification of the crude containing the compound of Formula (III) as defined above is required.

After removal of the protecting group of the compound of Formula (III) as defined above, i.e. after deprotection of the amino group, the amino keto-epoxide of Formula (II) as defined above, optionally in the form of a salt, can be reacted with the appropriate peptidic fragment to obtain the final pharmaceutically active compound, such as carfilzomib (when R is (CH₃)₂CHCH₂-), or other protease inhibitor. Methods for carrying out such reaction are well known in the art (cf. M. Elofsson, *ibid*.).

Particularly, the reaction of the salt of compound of Formula (II) as defined above wherein R is (CH₃)₂CHCH₂- with an amino acid chain of Formula (V) 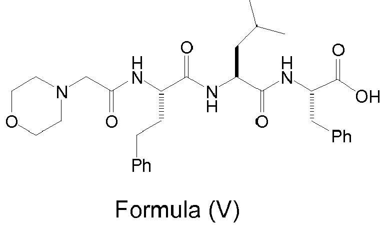 allows obtaining carfilzomib. Accordingly, another particular embodiment relates to a process for the preparation of carfilzomib, or a pharmaceutically acceptable salt thereof, comprising the following steps:
(i) preparing a compound of Formula (IIIa) 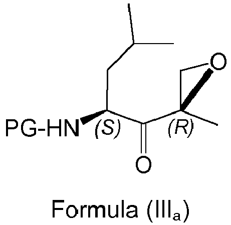 wherein PG is a protecting group selected from the group consisting of *tert-*butoxycarbonyl (Boc) and benzyloxycarbonyl (Cbz), by the process as defined above,
(ii) deprotecting the amine in the presence of a suitable acid, as explained above, particularly of trichloroacetic acid;
(iii) reacting the compound obtained in step (ii) with a compound of Formula (V) 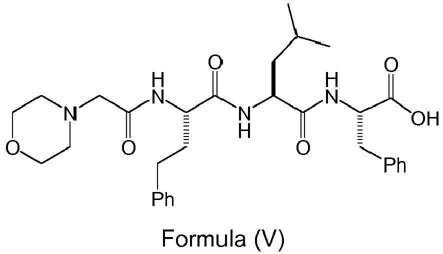 to obtain carfilzomib, or a pharmaceutically acceptable salt thereof; and optionally converting carfilzomib into a pharmaceutically acceptable salt thereof by reaction of the corresponding base with an appropriate pharmaceutically acceptable acid, or converting a carfilzomib salt into another carfilzomib salt by ion exchange.

Particularly, deprotection of step (ii) is carried out in the presence of trichloroacetic acid. Also particularly, step (iii) is carried out with O-(7-azabenzotriazol-1-yl)-1,1,3,3-tetramethyluronium hexafluorophosphate (HATU) and diisopropylethylamine (DIEA) in dimethylformamide (DMF).

The preparation of pharmaceutically acceptable salts can be carried out by methods known in the art. For example, they can be prepared from the parent compound which contains a basic or acidic moiety, by conventional chemical methods. Generally, such salts are prepared, for example, by reacting the free acid or base form of these compounds with a stoichiometric amount of an appropriate pharmaceutically acceptable base or acid in water, or in an organic solvent, or a mixture thereof.

The present invention covers all possible combinations of particular and preferred groups described herein.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration and are not intended to be limiting of the present invention. Furthermore, the present invention covers all possible combinations of particular and preferred embodiments described herein.

### Examples

In the following examples, products obtained using the process of the invention were characterized using the following analytical techniques:
1 H and 13C nuclear magnetic resonance spectra were collected at a 1 H resonance frequency of 400 MHz with a Varian Mercury spectrometer (equipped with a broadband probe ATB 1 H/19F/X of 5 mm). Chemical shifts were calibrated internally to the residual signal of the solvent in which the sample was dissolved (CDCl₃, δH 7.26, δC 77.0, CD₃OD, δH 4.89). Spectra were acquired dissolving 5-10 mg of sample in 0.6 mL of deuterated solvent. Mass spectra were obtained on a Agilent Technologies 6110 Quadrupole LC/MS spectrometer. High-performance liquid chromatography was carried out using a HP1100 Agilent Technologies spectrometer equipped with a UV detector. Gas chromatography was carried out using a 6890N GC-FID. XRPD analyses were performed using a PANalytical X'Pert diffractometer with Cu-K_{α} radiation in Bragg-Brentano geometry. The system is equipped with a monodimensional, real time multiple strip detector. Diffractograms were recorded from 3° to 40° (2θ) at a scan rate of 17.6° per minute. Optical rotations were measured on a JASCO P-2000 polarimeter.

### EXAMPLE 1. Preparation of tert-butyl ((S)-4-methyl-1-((R)-2-methyloxiran-2-yl)-1-oxopentan-2-yl)carbamate

To a solution of *tert-*butyl (*S*)-(2,6-dimethyl-3-oxohept-1-en-4 yl)carbamate (10 g, 37.2 mmol) in MeOH (80 mL) cooled to -10 °C, benzonitrile (8.4 mL, 82 mmol), hydrogen peroxide-urea adduct (14 g, 149 mmol) and tetramethylammonium hydroxide (6.9 g, 37.2 mmol) were added dropwise. The reaction mixture was stirred at -10 °C under N₂ atmosphere for 20 h. After dilution with a saturated Na₂S₂O₃ solution (120 mL) and 1 M HCl (7 mL), MeOH was evaporated under reduced pressure and the aqueous phase was extracted with EtOAc (50 mL x 3). The combined organic phases were dried over MgSO₄ and concentrated in vacuo affording a crude which was suspended in toluene (40 mL), stirred at 0-5 °C for 1 h, and filtered. The resulting solution was concentrated in vacuo affording a crude oil (7.45 g) containing *tert*-butyl((*S*)-4-methyl-1-((*R*)-2-methyloxiran-2-yl)-1-oxopentan-2-yl)carbamate (diastereomeric ratio (dr)>10:1 by ¹H-NMR) as the main product. This crude was purified by flash chromatography (cyclohexane/ethyl acetate) affording *tert-*butyl ((*S*)-4-methyl-1-((*R*)-2-methyloxiran-2-yl)-1-oxopentan-2-yl)carbamate (4.04 g, 40% yield) as an oil.

¹H-NMR (400 MHz, CDCl₃) d 4.84 (d, *J* = 8 Hz, 1 H), 4.32 (ddd, J = 12, 10.4 and 3.2 Hz, 1 H), 3.29 (d, *J* = 4.8 Hz, 1 H), 2.88 (d, *J* = 4.8 Hz, 1 H), 1.72 (m, 1 H), 1.49 (s, 3 H), 1.45 (m, 1 H), 1.17 (ddd, *J =* 14.4, 10.4 and 4.2 Hz, 1 H), 0.97 (d, *J =* 6.8 Hz, 3 H), 0.93 (d, *J* = 6.8 Hz, 3 H).
Analysis GC (optical purity) 96% ee

### Example 2. Preparation of benzyl ((S)-4-methyl-1-((R)-2-methyloxiran-2-yl)-1-oxopentan 2-yl)carbamate

To a solution of (*S*)-benzyl (2,6-dimethyl-3-oxohept-1-en-4-yl)carbamate (0.2 g, 0.69 mmol) in MeOH (1.6 mL) cooled to -10 °C, benzonitrile (0.15 mL, 1.52 mmol), hydrogen peroxide-urea adduct (0.26 g, 2.76 mmol) and tetrabutylammonium hydroxide (0.51 mL, 0.69 mmol) were added dropwise. The reaction mixture was stirred at -10 °C under N₂ atmosphere for 20 h. After dilution with a saturated Na₂S₂O₃ solution (2 mL) and 1 M HCl (drops), MeOH was evaporated under reduced pressure and the aqueous phase was extracted with EtOAc (5 mL x 3). The combined organic phases were dried over MgSO₄ and concentrated in vacuo affording a crude which was purified by flash chromatography (cyclohexane/ethyl acetate) affording benzyl ((*S*)-4-methyl-1-((*R*)-2-methyloxiran-2-yl)-1-oxopentan-2-yl)carbamate (51 mg, 24% yield).

¹H-NMR (400 MHz, CDCl₃) d 7.34 (m, 5H), 5.04 (m, 1 H), 5.05 (d, *J =* 5.2 Hz, 2 H), 4.40 (ddd, J = 11.6, 10.8 and 2.8 Hz, 1 H), 3.27 (d, *J* = 4.8 Hz, 1 H), 2.90 (d, *J =* 5.2 Hz, 1 H), 1.72 (m, 1 H), 1.54 (s, 3 H), 1.52 (m, 1 H), 1.21 (m, 1 H), 0.97 (d, *J* = 6.4 Hz, 3 H), 0.93 (d, *J = 6.8* Hz, 3 H).

### Example 3. Preparation of tert-butyl ((S)-4-methyl-1-((R)-2-methyloxiran-2-yl)-1-oxopentan-2-yl)carbamate

To a solution of *tert-*butyl (*S*)-(2,6-dimethyl-3-oxohept-1-en-4 yl)carbamate (1 g, 3.72 mmol) in MeOH (8 mL) cooled to -10 °C, benzonitrile (0.84 mL, 8.2 mmol), 30% aqueous hydrogen peroxide solution (1.52 mL, 14.9 mmol) and tetramethylammonium hydroxide (0.69 g, 3.72 mmol) were added dropwise. The reaction mixture was stirred at -10 °C under N₂ atmosphere for 20 h. After dilution with a saturated Na₂S₂O₃ solution (12 mL) and 1 M HCl (2 mL), MeOH was evaporated under reduced pressure and the aqueous phase was extracted with EtOAc (5 mL x 3). The combined organic phases were dried over MgSO₄ and concentrated in vacuo affording a crude (dr >15:1 by ¹H NMR) which was purified by flash chromatography (cyclohexane/ethyl acetate) to give *tert-*butyl ((*S*)-4-methyl-1-((*R*)-2-methyloxiran-2-yl)-1-oxopentan-2-yl)carbamate (0.37 g, 37% yield) as an oil.

### Example 4. Preparation of (S)-2-amino-4-methyl-1-((R)-2-methyloxiran-2-yl)pentan-1-one trichloroacetic acid salt

To a solution of *tert-*butyl (*S*)-(2,6-dimethyl-3-oxohept-1-en-4 yl)carbamate (2.6 g, 10 mmol) in MeOH (20.6 mL) cooled to -10 °C, benzonitrile (2.3 mL, 22 mmol), hydrogen peroxide-urea adduct (3.8 g, 40.3 mmol) and tetramethylammonium hydroxide (1.9 g, 10 mmol) were added dropwise. The reaction mixture was stirred at -10 °C under N₂ atmosphere for 20 h. After dilution with a saturated Na₂S₂O₃ solution (30 mL), MeOH was evaporated under reduced pressure and the aqueous phase was extracted with EtOAc (15 mL x 3). The combined organic phases were dried over MgSO₄ and concentrated in vacuo affording a residue which was suspended in toluene (10 mL), stirred at 0-5 °C for 1 h and filtered. The resulting solution was concentrated in vacuo affording a crude oil (1.8 g) containing, as major product, *tert*-butyl ((*S*)-4-methyl-1-((*R*)-2-methyloxiran-2-yl)-1-oxopentan-2-yl)carbamate (dr >10:1 by ¹H-NMR).

To a solution of this crude oil (0.59 g) in CH₂Cl₂ (3 mL), trichloroacetic acid (1.41 g, 8.6 mmol) was added. The resulting solution was stirred at room temperature under N₂ overnight. The reaction mixture was concentrated in vacuo affording a crude oil. To this oil, a solution of heptane/TBME (1:1, 3.5 mL) was added with stirring. The mixture was stirred for 1 h at room temperature and for another 1 h at 0-5 °C. The resulting solid was filtered and washed with cold heptane/TBME (1:1, 3 x 0.5 mL), and then dried under vacuum at room temperature to give (*S*)-2-amino-4-methyl-1-((*R*)-2-methyloxiran-2-yl)pentan-1-one trichloroacetic acid salt (0.21 g, 30% yield, dr >28:1 by ¹H-NMR) as a white solid.
¹H-RMN (400 MHz, CD₃OD) δ 4.06 (dd, *J* = 10 and 2.8 Hz, 1 H), 3.13 (d, *J* = 4.4 Hz, 1 H), 3.03 (d, *J* = 4.4 Hz, 1 H), 1.76 (m, 2 H), 1.49 (t, *J =* 10 Hz, 1 H), 1.01 (d, *J =* 3.6 Hz, 3 H), 0.99 (d, *J* = 4.0 Hz, 3 H).
Optical rotation (MeOH, c = 1, 25 °C) +70.6.
XRPD 8.0, 8.9, 13,6, 15.1, 16.2, 17.9, 20.9, 22.4, 22.6, 23.5, 24.4, 24.6, 26.9, 28.8, 32.4, 32.7° ± 0.2 ° Theta (Cu-K_{α} radiation λ = 1.5406 Å). See Fig. 1.

### Example 5. Preparation of (S)-2-amino-4-methyl-1-((R)-2-methyloxiran-2-yl)pentan-1-one trichloroacetic acid salt

To a solution of *tert*-butyl (*S*)-(2,6-dimethyl-3-oxohept-1-en-4 yl)carbamate (4 g, 14.9 mmol) in MeOH (32 mL) cooled to -10 °C, benzonitrile (3.38 mL, 32.7 mmol), hydrogen peroxide-urea adduct (5.6 g, 59.5 mmol) and tetramethylammonium hydroxide (2.78 g, 14.9 mmol) were added dropwise. The reaction mixture was stirred at -10 °C under N₂ atmosphere overnight. After dilution with a saturated Na₂S₂O₃ (48 mL) and HCl 1 M (3 mL), MeOH was evaporated under reduced pressure and the aqueous phase was extracted with AcOEt (15 mL x 3). The combined organic phases were dried over MgSO₄ and concentrated in vacuo affording a residue which was suspended in toluene (16 mL), stirred at 0-5 °C for 1 h, and filtered. The solution was concentrated in vacuo affording a crude oil containing *tert-*butyl ((*S*)-4-methyl-1-((*R*)-2-methyloxiran-2-yl)-1-oxopentan-2-yl)carbamate (2.7 g, 67% yield, dr >8:1 by ¹H-NMR) as the major product.

To a solution of this crude (1.18 g, 4.37 mmol) in toluene (6 mL), trichloroacetic acid (3.56 g, 21.8 mmol) was added. The resulting solution was stirred at room temperature under N₂ overnight. The reaction mixture was concentrated in vacuo affording a crude oil. To this oil a mixture of heptane/TBME(1:1, 4.5 mL) was added with stirring. The mixture was stirred for 1 h at room temperature and for a further 1 h at 0-5 °C. The resulting solid was filtered, washed with cold heptane/TBME (1:1, 3 x 0.3 mL), and dried under vacuum at room temperature to give (*S*)-2-amino-4-methyl-1-((*R*)-2-methyloxiran-2-yl)pentan-1-one trichloroacetic acid salt (0.53 g, 36% yield, dr >29:1 by ¹H-NMR) as a white solid.

### Comparative Example 1. Preparation of (S)-2-amino-4-methyl-1-((R)-2-methyloxiran-2-yl)pentan-1-one trifluoroacetic acid salt

To the crude containing *tert*-butyl ((*S*)-4-methyl-1-((*R*)-2-methyloxiran-2-yl)-1-oxopentan-2-yl)carbamate obtained in Example 5 (0.5 g, 1.84 mmol), a mixture of CH₂Cl₂/TFA (3:1, 3.8 mL) was added. The resulting solution was stirred at room temperature for 30 min (TLC showed complete reaction). The solvent was removed in vacuo and the residue was coevaporated several times with TBME. The residue was stirred in TBME (1.5 mL) and heptane (3 mL) was added dropwise. The mixture was stirred at 0-5 °C for 30 min but no solid was observed. After stirring overnight at 0-5 °C no solid was observed.

### Comparative Example 2

Ca(OCl)₂ (0.75 g, 5.24 mmol) was slowly added to H₂O (1.5 mL) at 0 °C. The mixture was cooled to -5 to -10 °C and was stirred for 10 min. *N*-methyl-2-pyrrolidone (7 mL) was added and the mixture was stirred for 15 min. *tert*-Butyl (*S*)-(2,6-dimethyl-3-oxohept-1-en-4-yl)carbamate (0.33 g, 1.3 mmol) dissolved in *N*-methyl-2-pyrrolidone (2.8 mL) was added dropwise and the reaction mixture was stirred for 4 h at 0-5 °C. After this time, 1 M aqueous Na₂SO₃ (3.5 mL) was added and the mixture was extracted with AcOEt. The combined organic phases were washed with H₂O and with saturated aqueous NaCl solution, dried over MgSO₄ and concentrated under reduced pressure to give the crude product (0.26 g) as an orange oil. This residue was filtered through SiO₂ eluting with cyclohexane/AcOEt (from 100/0 to 95/5 cyclohexane/AcOEt) to give *tert-*butyl ((*S*)-4-methyl-1-((*R*)-2-methyloxiran-2-yl)-1-oxopentan-2-yl)carbamate (0.19 g, diastereomeric ratio 2.4:1 (*S,R*:*S,S*) by GC) as a yellow oil

### Citation List

### Patent Literature:

- 1.: WO2006017842
- 2.: WO2005105827
- 3.: WO2009045497
- 4.: WO2005111009
- 5.: WO201136905
- 6.: WO2007149512

### Non Patent Literature:

7. N. Sin, et al. "Total synthesis of the potent proteasome-inhibitor epoxomicin: a useful tool for understanding proteasome biology", Bioorg. Med. Chem. Lett. 1999, Vol. 9, pp. 2283-2288.
8. M.Elofsson, et al. "Towards subunit-specific proteasome inhibitors: synthesis and evaluation of peptide alpha', beta'-epoxyketones" Chemistry & Biology, 1999, Vol 6, pp.811-822
9. TW Green et al., "Protective Groups in Organic Chemistry", chapter 7, "Protection for the amino Group ", Wiley, 3rd ed. 1999, pp. 503-550

## Claims

1. A compound which is the trichloroacetic acid salt of a compound Formula (II) 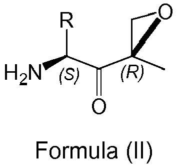 wherein R is selected from the group consisting of H, CH₃-, (CH₃)₂CH-, (CH₃)₂CHCH₂-, CH₃CH₂CH(CH₃)-, and PhCH₂-.

2. The compound according to claim 1, wherein R is (CH₃)₂CHCH₂- which is (*S*)-2-amino-4-methyl-1-((*R*)-2-methyloxiran-2-yl)pentan-1-one trichloroacetic acid salt (IIₐ).

3. The compound according to claim 2 which is in a crystalline form.

4. The compound according to claim 3, wherein the crystalline form is **characterized by** the main peaks in its powder X-ray diffraction pattern obtained using copper K-alpha₁ radiation comprising diffraction peaks at approximately 8.0, 8.9, 13,6, 15.1, 16.2, 17.9, 20.9, 22.4, 26.9, 28.8 ± 0.2 degrees 2-theta.

5. A process for the preparation of the trichloroacetic acid salt of a compound Formula (II) 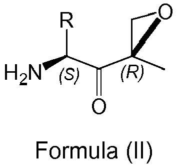 wherein R is selected from the group consisting of H, CH₃-, (CH₃)₂CH-, (CH₃)₂CHCH₂-, CH₃CH₂CH(CH₃)-, and PhCH₂-;
comprising deprotecting the amino group of a compound of Formula (III) 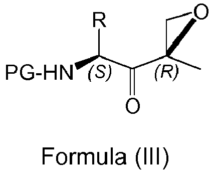 wherein R is as defined above, and PG is a protecting group selected from the group consisting of *tert*-butoxycarbonyl (Boc) and benzyloxycarbonyl (Cbz);
wherein the deprotection is carried out in the presence of the necessary amount of trichloroacetic acid to form the salt.

6. The process according to claim 5, wherein R is (CH₃)₂CHCH₂- and the process further comprises:
a) crystallizing (*S*)-2-amino-4-methyl-1-((*R*)-2-methyloxiran-2-yl)pentan-1-one trichloroacetic acid salt from a solution of said compound in a solvent system comprising a solvent selected from the group consisting of *tert-butyl* methyl ether (TBME), TBME/heptane, or toluene;
b) isolating the crystalline form of (*S*)-2-amino-4-methyl-1-((*R*)-2-methyloxiran-2-yl)pentan-1-one trichloroacetic acid salt obtained in the prior step; and
c) removing the solvent from the crystalline form of (*S*)-2-amino-4-methyl-1-((*R*)-2-methyloxiran-2-yl)pentan-1-one trichloroacetic acid salt obtained in step b), to yield the crystalline form of the compound (*S*)-2-amino-4-methyl-1-((*R*)-2-methyloxiran-2-yl)pentan-1-one trichloroacetic acid salt (IIa) as defined in claim 4.

7. The process according to claims 5 or 6, further comprising a previous step of reacting a compound of Formula (IV) 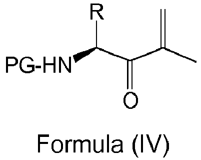 wherein R is selected from the group consisting of H, CH₃-, (CH₃)₂CH-, (CH₃)₂CHCH₂-, CH₃CH₂CH(CH₃)-, and PhCH₂-; and PG is a protecting group selected from the group consisting of *tert*-butoxycarbonyl (Boc) and benzyloxycarbonyl (Cbz),
with benzonitrile and either aqueous H₂O₂ or H₂O₂-urea in the presence of from 0.5 to 1 equivalents of a tetra(C₁-C₆)alkylammonium hydroxide as a base, in a polar solvent system, and at a suitable temperature, in order to obtain the compound of Formula (III).

8. A process for the preparation of a compound of Formula (III) 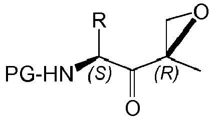 Formula (III)
wherein
R is selected from the group consisting of H, CH₃-, (CH₃)₂CH-, (CH₃)₂CHCH₂-, CH₃CH₂CH(CH₃)-, and PhCH₂-;
PG is a protecting group selected from the group consisting of *tert*-butoxycarbonyl (Boc) and benzyloxycarbonyl (Cbz);
the process comprising the stereoselective epoxidation of compound of Formula (IV) 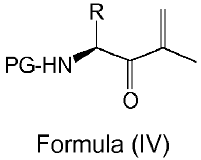 wherein R and PG are as defined above, by reaction with benzonitrile and either aqueous H₂O₂ or H₂O₂-urea in the presence of from 0.5 to 1 equivalents of a tetra(C₁-C₆)alkylammonium hydroxide as a base, in a polar solvent system, and at a suitable temperature.

9. The process according to claim 8, further comprising deprotecting the amino group of the compound of Formula (III) to obtain a salt of the amino keto-epoxide of Formula (II) 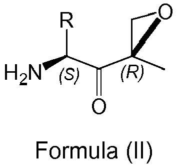 wherein R is selected from the group consisting of H, CH₃-, (CH₃)₂CH-, (CH₃)₂CHCH₂-, CH₃CH₂CH(CH₃)-, and PhCH₂-;
wherein the deprotection is carried out in the presence of a necessary amount of an acid selected from the group consisting of trifluoroacetic acid, and trichloroacetic acid to yield the corresponding salt.

10. The process according to any one of claims 7 to 9, wherein the polar solvent system is selected from the group consisting of methanol, ethanol, propanol, isopropyl alcohol, butanol, *tert*-butanol, dimethylformamide, tetrahydrofuran, dimethylformamide, dimethylsulfoxide, acetonitrile, or a mixture thereof, optionally in the presence of water.

11. The process according to any one of claims 7 to 10, wherein the amount of benzonitrile is from 1 to 3 equivalents and the amount of H₂O₂ or H₂O₂-urea in the form of an aqueous solution is from 3 to 6 equivalents.

12. The process according to any one of claims 5 to 11, wherein PG is *tert*-butoxycarbonyl (Boc) or benzyloxycarbonyl (Cbz).

13. The process according to claims 5 to 12, wherein R is (CH₃)₂CHCH₂-.

14. The process according to any one of claims 5 to 7 or 9 to 12, further comprising reacting the compound as defined in any one of claims 1 to 3 wherein R is (CH₃)₂CHCH₂- or PhCH₂-, or a salt of compound of Formula (II) wherein R is (CH₃)₂CHCH₂- or PhCH₂-with a peptide compound to obtain a pharmaceutically active compound selected from 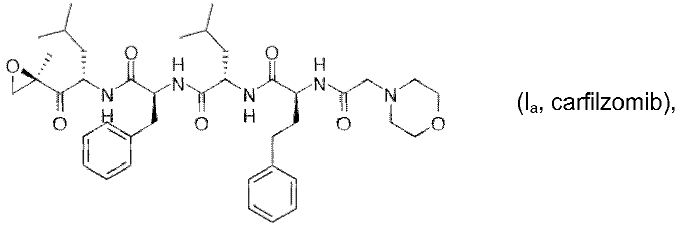 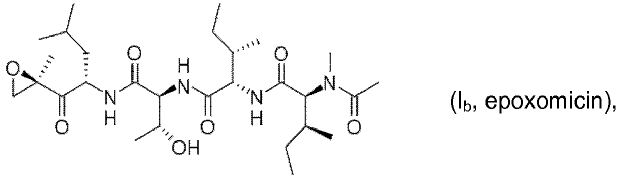 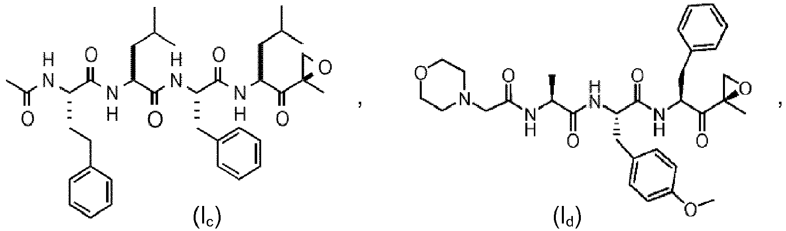 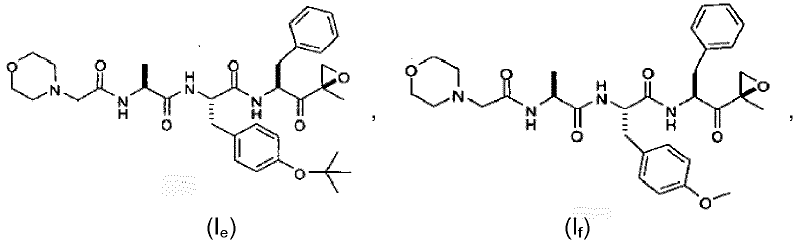 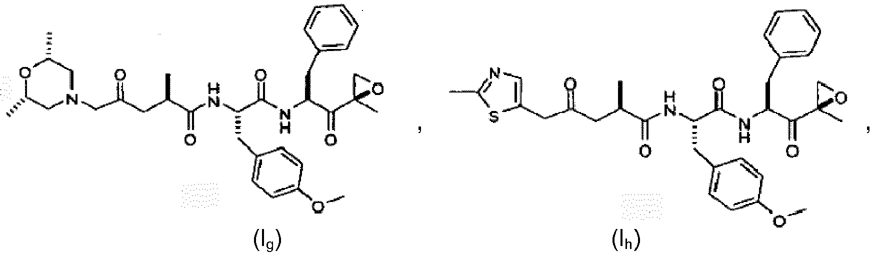 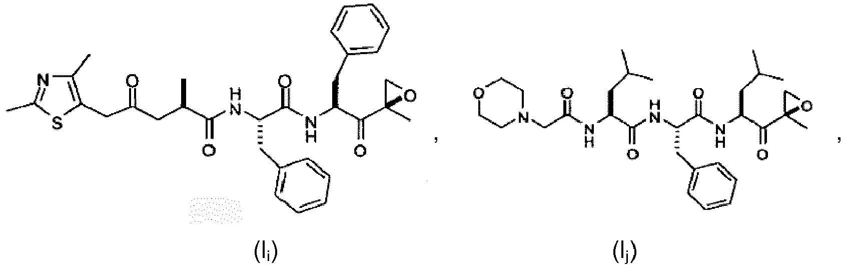 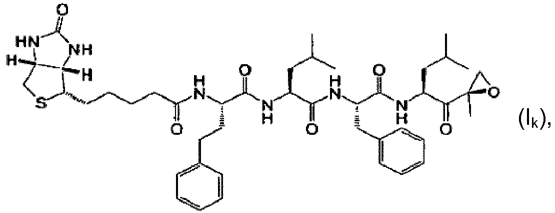 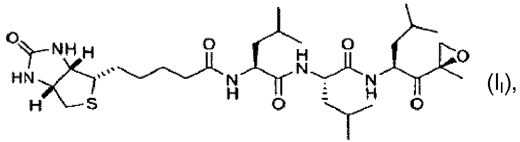 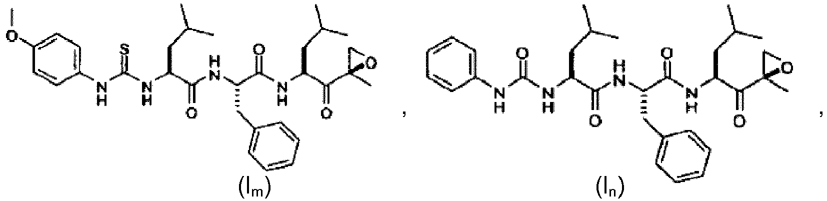 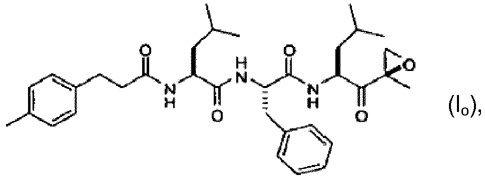 and 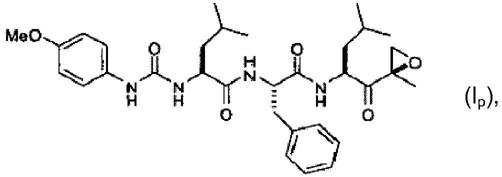 or a pharmaceutically acceptable salt thereof and, optionally, converting the pharmaceutically active compound into a pharmaceutically acceptable salt thereof by reaction of the corresponding base with an appropriate pharmaceutically acceptable acid, or converting a salt of the pharmaceutically active compound to another salt of the pharmaceutically active compound by ion exchange.

15. The process according to claim 14, wherein R is (CH₃)₂CHCH₂- and the peptide compound is a compound of Formula (V) 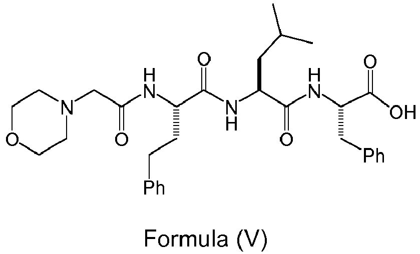 to obtain carfilzomib (Iₐ), or a pharmaceutically acceptable salt thereof.
